Europäisches Patentamt

European Patent Office

Office européen des brevets

(19)

(11) **EP 1 388 345 A1**

(12) **EUROPEAN PATENT APPLICATION**

(43) Date of publication:
**11.02.2004  Bulletin 2004/07**

(51) Int Cl.[7]: **A61L 15/42**

(21) Application number: **02447152.6**

(22) Date of filing: **09.08.2002**

| | |
|---|---|
| (84) Designated Contracting States:<br>**AT BE BG CH CY CZ DE DK EE ES FI FR GB GR IE IT LI LU MC NL PT SE SK TR**<br>Designated Extension States:<br>**AL LT LV MK RO SI** | (72) Inventors:<br>• **Mariani, Manuel**<br>  **65016 Montesilvano (Pescara) (IT)**<br>• **Corzani, Italo**<br>  **66100 Chieti (IT)** |
| (71) Applicant: **The Procter & Gamble Company**<br>**Cincinnati, Ohio 45202 (US)** | (74) Representative: **Canonici, Jean-Jacques et al**<br>**NV Procter & Gamble Services Company SA,**<br>**Temselaan 100**<br>**1853 Strombeek-Bever (BE)** |

(54) **Polymeric compositions with enhanced vapour permeability and washability**

(57)    The present invention relates to polymeric compositions for making a liquid impermeable, moisture vapour permeable layer by coating the composition onto a substrate. The polymeric compositions comprise preferred thermoplastic polymers and suitable hydrophilic plasticizers which are covalently bonded to said thermoplastic polymers. The layers made from the polymeric compositions of the present invention exhibit enhanced moisture vapour permeability and washability. These layers can find a variety of applications wherein moisture vapour permeability is desirable for example within waterproof garments, protective bedding covers, and within absorbent articles such as diapers, sanitary napkins, panty liners, incontinence products, protective clothing and the like.

EP 1 388 345 A1

Printed by Jouve, 75001 PARIS (FR)

**Description**

**Field of the Invention**

The present invention relates to polymeric compositions for making a moisture vapour permeable, liquid impermeable layer. The compositions of the present invention can find a variety of applications, wherein moisture vapour permeability and impermeability is desirable, in both durable and disposable articles, for example within waterproof garments, protective bedding covers, as well as absorbent articles such as diapers, sanitary napkins, panty liners, incontinence products, protective clothing and the like. The layers formed from the polymeric compositions of the present invention, exhibit an enhanced moisture vapour permeability and washability.

**Background of the Invention**

Protective garments for wear in rain and other wet conditions should keep the wearer dry by preventing the leakage of water into the garment and by allowing perspiration to evaporate form the wearer to the atmosphere. It is widely recognized that these protective garments for being comfortable, must be "breathable", i.e. they must be liquid impermeable but allow the passage of water vapour. However, it is not necessary that air pass through the garment for it to be comfortable, only that water vapor form perspiration be transmitted from one inside to outside so that the undergarments do not become wet and so that the natural evaporative cooling effect can be achieved. Typically, protective garments are obtained by covering suitable textile materials with a waterproof material having the desired properties.

Thermoplastic films that provide a liquid barrier in addition to providing moisture vapour permeability, are known in the art. Particularly preferred are hydrophilic continuous films that do not allow the flow of moisture vapour through open pores or apertures in the material, but do transfer substantial amounts of moisture vapour through the film by absorbing water on one side of the film where the moisture concentration is higher, and desorbing or evaporating it on the opposite side of the film where the moisture vapour concentration is lower. Such films are typically formed from a polymeric composition comprising a hydrophilic polymer, or a blend of hydrophilic polymers. Hydrophilic polymeric compositions having the above described characteristics are also known in the art as "monolithic compositions", and the moisture vapour permeable, liquid impermeable layers or films made therefrom are known as "monolithic layers" or "monolithic films".

For example WO 95/16746 discloses films prepared from mixtures of a) block copolyether ester, block copolyether amides (e.g. Pebax™) and or polyurethane and b) thermoplastic polymer which is incompatible with a, and c) a compatibiliser. The films are liquid impermeable and have a moisture vapour permeability of about 700 $g/m^2.day$.

Also, US Patent No. 5,447,783 discloses a vapour permeable water resistant multi component film structure having at least three layers. The outer layers are hydrophobic copolyether-ester elastomers having a thickness of 1.3-7.6 micrometers and a Water Vapour Transmission Rate (WVTR) of 400-2500 $g/m^2.day$ and the inner layer is a hydrophilic copolyether-ester elastomer having a thickness of 7.6-152 micrometers and a WVTR of at least 3500 $g/m^2.day$.

US Patent No. 4,493,870 discloses a flexible layered waterproof product comprising a textile material covered with a film of a copolyetherester having a Moisture Vapour Transmission Rate (MVTR) of at least 1000 $g/m^2.day$ (ASTM E96-66) having a thickness of 5 to 35 micrometers.

GB Patent No. 2024100 discloses a flexible layered water resistant article comprising a microporous hydrophobic outer layer which is moisture vapour permeable but resist liquids and a hydrophilic inner layer of polyetherpolyurethane having a MVTR of above 1000 $g/m^2.day$.

Compositions known for providing hydrophilic continuous moisture vapour permeable, liquid impermeable films or layers include thermoplastic polymers such as polyurethanes, poly-ether-amides block copolymers, polyethylene-acrylic acid copolymers, polyethylene oxide and its copolymers, poly lactide and copolymers, polyamides, polyester block copolymers, sulfonated polyesters, poly-ether-ester block copolymers, poly-ether-ester-amide block copolymers, polyacrylates, polyacrylic acids and derivatives, ionomers, polyethylene-vinyl acetate with a vinyl acetate content of more than 28% by weight, polyvinyl alcohol and its copolymers, polyvinyl ethers and their copolymers, poly-2-ethyl-oxazoline and derivatives, or mixtures thereof. Such compositions can be used for making layers and films featuring good values of moisture vapour permeability while being liquid impermeable.

In WO 99/64505, are disclosed thermoplastic compositions for making hydrophilic continuous moisture vapour permeable, liquid impermeable layers. The thermoplastic compositions comprising preferred thermoplastic polymers such as polyurethanes, are rendered readily processable by inclusion of a suitable plasticizer that lowers the viscosity of the compositions; the selection of hydrophilic plasticizers allows obtaining layers formed from these thermoplastic compositions, which exhibit an enhanced moisture vapour permeability if compared to a corresponding film or layer made from a composition not comprising the plasticizer. In this context, MVTR values of up to 500 $g/m^2.day$ have been

obtained.

The above-mentioned thermoplasic compositions require the use of intrinsically breathable thermoplastic polymers which need to be selected from defined species, there would be an advantage in proposing a breathable polymeric composition that can be comprised of a broader range of thermoplastic polymers.

Hence, it is an object of the present invention to provide a polymeric composition for making moisture vapour permeable, liquid impermeable layers that can also be formed from non- or low breathable thermoplastic polymers.

The solution provided by the present invention consists of rendering breathable said non- or low breathable thermoplastic polymers, through a reaction with hydrophilic plasticizers. As these hydrophilic plasticizers have the capacity to react with said thermoplastic polymers, they are called reactive plasticizers.

In WO 01/04224, are disclosed aqueous polymeric compositions comprising (A) a water-soluble component comprising at least one functional group that undergoes a crosslinking reaction, and (B) one film-forming polymer. The layers made from these compositions, are taught to provide protection from the effects of liquids, and they typically are non-breathable.

In addition, it is known that for certain applications and particularly for articles which are intended to come in contact with liquid water or which could come in contact with liquid water, such as for example waterproof garments or tents, it is critical that these articles exhibit a good washability, on top of satisfactory liquid impermeability and vapour permeability. This aspect is particularly important in textile coating for durable or semi-durable articles.

Therefore, it is another object of the present invention to provide compositions for making moisture vapour permeable, liquid impermeable layers with improved washability or durability. Such benefits are now derivable from the compositions of the invention, which provide the necessary immobilization of the plasticizers, through the reaction with the thermoplastic polymers, and thus prevent leakage of said hydrophilic plasticizers upon washing.

Other objects and more specific properties of the polymeric compositions according to the present invention will be clear after reading the following description of the invention.

## Summary of the Invention

In one embodiment, the present invention relates to a polymeric composition comprising the reaction product of:

    a thermoplastic polymer or blend of thermoplastic polymers, and
    a hydrophilic plasticizer or blend of hydrophilic plasticizers,

wherein at least one of said hydrophilic plasticizers is covalently bonded with at least one of said thermoplastic polymers.

In another embodiment of the present invention, is provided a process for making polymeric compositions according to the present invention, comprising the steps of:

    providing a thermoplastic polymer or blend of thermoplastic polymers, and
    providing a hydrophilic plasticizer or blend of hydrophilic plasticizers capable of covalently bonding with at least one of said thermoplastic polymers to form said polymeric compositions.

In still another embodiment, the present invention relates to a moisture vapour permeable layer formed from the polymeric compositions according to the present invention, wherein said layer is liquid impervious and has a water vapour transmission rate (WVTR) of at least 600 $g/m^2$.day, preferably at least 800 $g/m^2$.day, more preferably at least 1000 $g/m^2$.day, and most preferably at least 1300 $g/m^2$.day, measured with a thickness of said layer or film at 20 □m.

In yet another embodiment of the present invention, is provided a moisture vapour permeable, liquid impervious composite comprising at least one layer according to the previous embodiment coated onto a substrate, said substrate being moisture vapour permeable.

Further, the present invention refers to a moisture vapour permeable, liquid impervious article comprising at least one layer or composite according to the preceding embodiments.

## Detailed description of the invention

In a first embodiment, the present invention relates to a polymeric composition comprising the reaction product of:

    a thermoplastic polymer or blend of thermoplastic polymers, and
    a hydrophilic plasticizer or blend of hydrophilic plasticizers,

wherein at least one of said hydrophilic plasticizers is covalently bonded with at least one of said thermoplastic polymers.

As stated in the background of the invention, polymeric compositions for making films which provide a liquid barrier, in addition to moisture vapour permeability, are known in the art. Particularly suitable are hydrophilic continuous films that do not allow the flow of moisture vapour through the open pores or apertures in the material, but do transfer substantial amounts of moisture vapour through the film by absorbing water on one side of the film where the moisture vapour concentration is higher, and desorbing or evaporating it in the opposite side of the film where the moisture vapour concentration is lower.

Polymers that can be used as thermoplastic polymers according to the present invention, may be selected from intrinsically breathable monolithic polymers and, according to a particular advantage of the present invention, also from non- or low breathable thermoplastic polymers.

Suitable thermoplastic polymers comprised in the composition according to the present invention, include polyurethanes, poly-ether-amides, polyethylene-acrylic acid copolymers, polyethylene oxide and its copolymers, poly lactide and copolymers, polyamides, polyester block copolymers, sulfonated polyesters, poly-ether-esters, poly-ether-ester-amides, polyacrylates, polyacrylic acids and derivatives, ionomers, polyethylene-vinyl acetate with a vinyl acetate content of more than 28% by weight, polyvinyl alcohol and its copolymers, polyvinyl ethers and their copolymers, styrene-maleic anhydride copolymers, poly-2-ethyl-oxazoline and derivatives, or mixtures thereof.

As mentioned in the background section, polymeric compositions comprising thermoplastic polymers may incorporate suitable plasticizers. The plasticizers can be added to alter a variety of properties of the composition including such properties as, e.g., extrudability, flexibility, workability or stretchability. Such compounds may be added, for example, to lowering the viscosity of the thermoplastic polymers at certain conditions.

According to the present invention, it has been surprisingly found that by selecting the hydrophilic plasticizer or blend of plasticizers such that at least one of said plasticizers is capable of covalently bonding with at least one of said thermoplastic polymers, the advantage of an enhanced moisture vapour permeability of the resulting layer or film formed from the polymeric composition is achieved, when compared to a corresponding film or layer formed from a polymeric composition comprising the same thermoplastic polymer, but without the plasticizer.

While in WO 99/64505, suitable plasticizers were physically linked to the polymer chain, the polymeric composition according to the present invention, comprises plasticizers and thermoplastic polymers that are covalently, i.e. chemically bonded. This particular feature has surprisingly allowed overcoming the problem of poor compatibility that was recurrently encountered in the prior art, while trying to combine non- or low hydrophilic polymers with very hydrophilic plasticizers.

According to the present invention, it has been allowed to form moisture vapour permeable, liquid impermeable layers from non- or low breathable thermoplastic polymers, i.e. which exhibit a maximum MVTR value of about 600 g/ $m^2$.day, at 20 micrometers. This particular class of polymers being much cheaper than the breathable polymers generally used in compositions for obtaining breathable films or layers, the present invention provides therefore, a low-cost alternative for producing said films or layers.

Suitable plasticizers according to the present invention should generaly comprise at least one functional group that forms a covalent bonding with the thermoplastic polymers. In a preferred embodiment of the present invention, the suitable plasticizer should contain one suitable functional group at each end of the plasticizer molecule, so that one of these functionalities react with one polymeric chain, while the other one could react with another polymeric chain. It is therefore possible to form a 3-dimensional network.

Preferred functional groups that undergo covalent bonding, comprise but are not limited to, hydroxyls, amines, epoxy, carboxyl groups, ketones, aldehydes, acrylates, or olefinically or acetylenically unsaturated groups. These chemical functionalities may be present both into the plasticizers, and into the thermoplastic polymeric chains, depending on the way they have been formed.

The covalent bonding between at least one of said plasticizers and at least one of said thermoplastic polymers, can be achieved by any suitable reaction known in the art Those skilled in the art will readily select the required chemical reaction. Particularly preferred are reactions of condensations, additions, nucleophile substitutions, or alkylations.

Preferred hydrophilic plasticizers, according to the present invention, are polyethylene glycol, polypropylene glycol, polytetramethylene glycol, and copolymers of thereof. Polyethylene glycol is particularly preferred.

Preferably the polymeric compositions of the present invention comprises from 10% to 80%, more preferably from 40% to 75% by weight of the polymeric composition, of the thermoplastic polymer or mixture of thermoplastic polymers, and from 20% to 90%, preferably from 25% to 60% by weight of the polymeric composition, of the suitable hydrophilic plasticizer or blend of hydrophilic plasticizers.

In a preferred aspect of the present invention, the aforementioned suitable hydrophilic plasticizer may be linked to said thermoplastic polymer by addition of a cross-linker.

The cross-linking between thermoplastic polymers, plasticizers, and cross-linkers creates a 3-dimensional matrix for the polymer. Physical cross-linking refers to polymers having crosslinks that are not chemical covalent bonds but are of a physical nature. Chemical cross-linking refers to polymers which are linked by covalent chemical bonds.

The cross-linker can be made to react by radiation techniques such as UV, E beam, gamma or micro-wave radiation or by co-polymerizing the monomers with a di/polyfunctional crosslinker via the use e. g., of UV, thermal and/ or redox polymerization initiators. The polymer can also be ionically crosslinked.

Suitable polyfunctional cross-linkers include, but are not limited to aminoplast (melamine/formaldehyde resins) such as UCB 376, Noveon AeroTex 3730, AeroTex 3030, phenoplast (phenol/formaldehyde resins), polyisocyanates, such as Baxenden Chemicals Trixene, or aziridine crosslinkers such as XAMA-2, XAMA-7.

Chemical crosslinking can also be achieved after polymerization by use of polyfunctional reagents capable of reacting with polymer functional groups such as ethyleneglycol diglycidyl ether, polyols such as glycerol, citric acid, and other polyfunctional reagents known in the art.

Crosslinking may also be effected all or in part by ionic crosslinking wherein groups of opposite charge interact via ionic interactions. Suitable ionic crosslinking agents include those known to the art including polyvalent cations such as $Al^{3+}$ and $Ca^{2+}$, di/poly-amines, di/poly-quaternary ammonium compounds, including polymeric polyamines and quaternary ammonium compounds known to the art.

In another aspect of the present invention, thermoplastic polymers or blend of polymers, when used in the molten state, may be highly viscous at the process conditions that are typical of the known processes of film or layer formation, e.g. an extrusion process involving a high power screw extruder. For example, they may have a viscosity higher than 5000 poise at a temperature of 20°C above the DSC (Differential Scanning Calorimetry) melting point, which is the temperature defined as that corresponding to the DSC peak, or corresponding to the highest DSC peak in case of a mixture of polymers showing more than one peak, and at a frequency of 1 rad/sec.

The viscosity of the preferred thermoplastic polymers or mixture of polymers can be adjusted by using the plasticizer, or blend of plasticizers of the present invention, that are capable of covalently bonding with the thermoplastic polymers and that lower the viscosity of the thermoplastic polymer or mixture of thermoplastic polymers in the melted state.

The compositions may be provided with at least one additional hydrophilic plasticizer, which is not capable of chemically bonding with the thermoplastic polymer or blend of thermoplastic polymers. It has been indeed surprisingly discovered that, in the polymeric compositions according to the present invention, the aforementioned suitable hydrophilic plasticizers show perfect compatibility with such non-reactive plasticizers, and actually facilitate the inclusion of further hydrophilic plasticizers in the compositions herein. This feature allows achieving a better processability of the compositions made with at least one non-reactive plasticizer, since the latter allow adjusting the viscosity of the preferred thermoplastic polymers or mixture of polymers.

Such non-reactive plasticizers comprise, but are not limited to, acetyl tributyl citrates, triacetin, citric acid esters, tartaric acid esters, glycerol and its esters, sorbitol, glycolates and mixtures thereof.

The polymeric compositions according to the present invention may also contain components which comprise at least two reactive groups capable of bridging said thermoplastic polymer and said hydrophilic plasticizer. Such components are required when the polymers and the plasticizers, according to the present invention, do not comprise functional groups that can directly react each other to form a covalent bonding. Therefore, the suitable component should be chosen depending upon the functionalities present on the two chemical actors. Examples of these components comprise but are not limited to PEG acrylic ester.

Additional compounds may also be present in the compositions of the present invention to improve the mechanical characteristics, as well as other characteristics such as tackiness, resistance to ageing by light and oxygen, visual appearance etc., of the films or layers formed from such polymeric compositions.

Such optional components include tackifying resins or blends of tackifying resins having a softening point of 125□C or less. Preferred resins, which may be present by up to 50% by weight of the polymeric compositions, may be selected form rosins and rosin esters, hydrocarbon resins, aliphatic resins, terpene and terpene-phenolic resins, aromatic resins, synthetic $C_5$ resins, mixtures of synthetic $C_5$-$C_9$ resins, and mixtures thereof.

Other optional components of said polymeric compositions include catalysts, activators, anti-oxidants, anti-ultraviolets, pigments, dyes, antibacterials, odour adsorbing materials, perfumes, pharmaceuticals, and mixtures thereof, which may be present within the composition at a level of up to 10% by weight of the composition.

In another embodiment of the present invention, is provided a process for making a polymeric composition according to the present invention, comprising the steps of:

   providing a thermoplastic polymer or blend of thermoplastic polymers, and
   providing a hydrophilic plasticizer or blend of hydrophilic plasticizers capable of covalently bonding with at least one of said thermoplastic polymers to form said polymeric composition.

It may be desirable to include in the above process, steps which will optimize said covalent bonding reaction; this comprises for example the addition of compounds such as crosslinkers, catalysts or activators, such like those described for the first embodiment of the present invention.

The process herein may involve at least two main alternative techniques, as described hereinafter. However, the practitioner can readily select any other suitable method for performing the individual steps described above.

In a preferred aspect of the present invention, the polymeric compositions may be obtained by using a solvent-based technique. In that case, a process for making the polymeric compositions according to the present invention typically comprise the steps of providing the thermoplastic polymer or mixture of polymers and the suitable plasticizer or blend of plasticizers, dissolving said components in a suitable solvent and compounding them, e.g. with a known suitable low shear or high shear mixer to form the polymeric composition in solution.

The polymeric compositions, according to the present invention, may be prepared either as homogeneous solution, if both of the components are soluble in the selected solvent; or as dispersion or emulsion, if one or both of these components are not soluble in the chosen solvent. In the latest case, it may be indeed required to effect a pre-emulsification or a dispersion of this compound by using techniques well known in the art.

In a preferred embodiment of the present invention, the polymeric compositions are prepared in the form of a waterborne system obtained by mixing either waterborne emulsions of polymers and plasticizers, solutions of polymers and emulsions of plasticizer, or polymer waterborne emulsions and solutions of plasticizers. The latest combination is particularly preferred.

In another aspect of the present invention, the polymeric compositions may be obtained by using hot melt techniques. A process for obtaining the claimed polymeric compositions will then typically comprise the steps of providing the thermoplastic polymer or mixture of polymers and the suitable plasticizer or blend of plasticizers, heating the components and compounding them, e.g. with a known suitable mixer to form the polymeric composition in the molten state having the desired complex viscosity $\eta^*$.

The polymeric compositions of the present invention comprising the preferred hydrophilic plasticizers or blend of plasticizers have the following complex viscosities ($n^*$):

50 poise $< \eta^* <$ 4000 poise, preferably 100 $< \eta^* <$ 200 poise, more preferably 100 $< n^* <$ 1000 poise, at a frequency of 1 rad/s at processing temperature (T) and $\eta^* <$ 2000 poise, preferably $\eta^* <$ 1000 poise, more preferably $\eta^* <$ 500 poise, at a frequency of 100 rad/s at processing temperature (T), wherein $\eta^*$ represents the complex viscosity of the polymeric composition. Preferably the temperature T is 190□C or less, more preferably 180□C or less and most preferably from 190□C to 70□C.

According to another embodiment of the present invention, a moisture vapour permeable, liquid impervious layer can be formed from the polymeric composition of the present invention. The films or layers formed from the polymeric compositions of the present invention preferably have breathability values, expressed as moisture vapour transport rate (MVTR) and measured according to the Moisture Vapour Transmission Test described herein, of at least 600 g/m$^2$.day, preferably at least 800 g/m$^2$.day, more preferably at least 1000 g/m$^2$.day, and most preferably at least 1300 g/m$^2$.day, with a thickness of said film or layer of at least 20 □m.

A moisture vapour permeable, liquid impervious layer can be formed from the polymeric composition of the present invention by coating said polymeric composition onto a substrate by any suitable means. Those skilled in the art can readily select the required application system.

While using solvent techniques, a process for making a layer or film from a polymeric composition according to the present invention typically comprises the steps of providing said composition in solution or dispersion, coating said composition in solution or dispersion onto a substrate, and evaporating said solvent or dispersant so that a layer having the desired thickness could be obtained.

After application to the substrate, compositions may be preferably subjected to accelerated drying and curing. Whereas the covalent reaction between the plasticizers and the polymers may already be initiated at room temperature, the above-mentioned curing step, at high temperature or by UV irradiation, allows this reaction to proceed rapidly and as completely as possible.

The polymeric compositions according to the present invention, and made using solvent techniques, may be applied by conventional means, including air or airless spray application, brushing, roller application, and the like. Spraying technique is particularly preferred.

The polymeric compositions made using hot melt techniques and having the complex viscosity above-described, allow for a film or layer to be coated onto a substrate using typical coating conditions and apparatuses known in the art for coating of low viscosities hot melt compositions in a layer having a required thickness onto a substrate, while also keeping the advantageous characteristics of the preferred thermoplastic polymers in providing hydrophilic continuous moisture vapour permeable, liquid impermeable layers or films. The polymeric compositions having such viscosities can also provide very thin films or layers.

A process for making a layer or film from a polymeric composition according to the present invention, and made using hot melt techniques, typically comprises the steps of providing said composition, heating it to make it flowable, and coating said composition in the molten state onto a substrate in a layer having the desired thickness.

In still another embodiment of the present invention, is provided a moisture vapour permeable, liquid impervious composite comprising at least one layer according to the previous embodiment coated onto a substrate.

While said substrate can be simply a formation substrate, onto which the polymeric composition is coated in order to form a film or layer of the desired thickness which is subsequently separated from said substrate and used as such, in one embodiment of the present invention a moisture vapour permeable, water impervious composite can also be formed which comprises the polymeric composition and a substrate onto which said polymeric composition is coated, wherein the substrate is also preferably moisture vapour permeable.

Such embodiment of the present invention provides a moisture vapour permeable, liquid impervious composite wherein the contribution of the layer formed from the polymeric composition of the present invention to the performance of the composite material resides only in the provision of a liquid barrier and hence could be advantageously provided as thinly as possible. The remaining performance physical criterion being preferably provided by the provided substrate, that therefore preferably acts also as a support layer.

The substrate, or support layer may be any useful layer which is preferably also moisture vapour permeable, preferably having a moisture vapour permeability of at least 600 $g/m^2$.day, preferably at least 800 $g/m^2$.day, more preferably at least 1000 $g/m^2$.day, and most preferably at least 1300 $g/m^2$.day.

Suitable substrates for use herein as support layers include two dimensional, planar micro and macro-porous films; macroscopically expanded films; formed apertured films; nonwoven and woven layers. According to the present invention, the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable two-dimensional porous planar layers of the backsheet may be made of any material known in the art, but are preferably manufactured from commonly available polymeric materials. Suitable materials are for example Goretex™ or Sympatex™ type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term two dimensional planar layer refers to layers having a depth of less than 1 mm, preferably less than 0.5 mm, wherein the apertures have an average uniform diameter along their length and which do not protrude out of the plane of the layer. The apertured materials for use as a backsheet in the present invention may be produced by using any of the methods known in the art as described in EPO 293 482 and the references therein. In addition, the dimensions of the apertures, produced by this method, may be increased by applying a force across the plane of the backsheet layer (i.e. stretching the layer).

Suitable macroscopically expanded films for use herein include films as described in for example in US Patent No. 4,637,819 and US Patent No. 4,591,523.

Preferred support layers for use herein include woven and nonwoven layers, most preferably hydrophobic fibrous layers such as hydrophobic nonwoven. Substrates such as fabrics and textiles are of particularly interest.

The composites of this preferred embodiment of the present invention are particularly advantageous as they allow the possibility of providing a composite wherein the polymeric composition may be coated onto the support substrate as a layer with the desired thickness. Typical coating conditions and apparatuses known in the art for the direct coating of low viscosities hot melts can be readily utilized in order to provide the polymeric composition at the desired thickness.

At least at the coating temperature, the polymeric composition in form of a layer preferably exhibits adhesive properties on the supportive substrate in order to form the preferred composite such that no additional adhesive is required to achieve a permanent attachment of the polymeric composition on the substrate. In some applications, it may be also desirable that the polymeric composition remains tacky at any temperature i.e. it is formulated so to have the typical characteristics of a pressure sensitive adhesive.

However, while solvent-based techniques as well as hot melt techniques have been described herein, any other known method for processing polymeric compositions can be used for processing the polymeric composition according to the present invention in any known form, i.e. films, layers, fibers, beads, filaments, stripes, etc. Also, any known method for spraying or foaming thermoplastic compositions can be used.

In a further embodiment, the present invention refers to a moisture vapour permeable, liquid impervious article comprising at least one layer or composite according to the present invention.

The polymeric compositions of the present invention and the moisture vapour permeable, liquid impervious layers and composites formed therefrom find utility in a number of applications wherein liquid imperviousness and moisture vapour permeability are desirable. The layers according to the present invention may be incorporated in many durable or disposable articles, including shape-formed, three-dimensional articles.

In particular, the present invention can be effectively utilized within waterproof garments. Are particularly contemplated, articles such as ski jackets, ski boots, trekking and hiking clothing, rainwear, water sports clothing, impermeable socks, tents, sleeping bags, and the like.

The present invention can also find utility within absorbent articles such as diapers, sanitary napkins, panty liners and incontinence products; perspiration pads such as underarm-, wrist- and head perspiration pads, collar inserts, shoe inserts, hat bands and brest pads; protective bedding covers, protective clothing, and the like.

A moisture vapour permeable, liquid impervious composite structure formed by coating the polymeric composition of the present invention onto a suitable substrate finds particular utility as the backsheet for absorbent articles especially sanitary napkins and panty liners. Such articles will typically comprise components known to the skilled person such as a liquid pervious topsheet, an absorbent core and backsheet and may optionally comprise fastening means, wings, and the like.

On top of the previously mentioned benefits, it has been surprisingly discovered that the polymeric compositions according to the present invention, when coated onto a substrate, exhibit an unexpected higher resistance to plasticizer wash out, i.e. they can withstand a large number of wash out cycles without substantive plasticizer loss due to water extraction. This property, also defined as washability or durability, is particularly critical for articles which are intended to come in contact with liquid water or which could come in contact with liquid water. Such articles are indeed particularly contemplated in the present invention.

Although the very hydrophilic character of the suitable plasticizers, tests for extractable matter performed with the polymeric compositions of the present invention, showed weight loss values below 0.5% of the total blend of reactive plasticizer that can be extracted.

Without being bound by theory, it is thought that by covalently bonding said plasticizer into said thermoplastic polymer, the latter is better locked into the polymeric matrix, which lead to a better durability, as well as a better stable formulation provided by the cross-linking.

As it can be seen with the above-mentioned advantages, some unexpectedly superior properties are provided by the compositions according to the present invention. It is undeniable that these surprising benefits derive from the covalent bonding between the hydrophilic plasticizer and the thermoplastic polymer. This particular feature indeed allows obtaining good compatibility between very hydrophilic plasticizers and non- or low hydrophilic polymers, while avoiding the drawback of plasticizer loss due to water extraction.

According to the present invention, the complex viscosity is measured using a Rheometer RDA-II available from Rheometrics Co. Moisture vapour permeability is measured as Water Vapour Transmission Rate (WVTR) at 23°C according to the ASTM E-96 "Upright Cup" method.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

### TEST METHODS

### 1. Moisture Vapour Transmission Test (MVTR)

Breathability of the materials of the present invention is intended to be measured as Moisture Vapour Transmission Rate at 23°C and 50% relative humidity according to the modified ASTM E-96 "Upright Cup" method. The only modification to the standard ASTM E-96 "Upright Cup" method consists in a change in the height of the air gap between the sample and the water surface in the cup, which height is 4 mm ± 0.5 mm, instead of 19 mm ± 2.5 mm, as specified in the standard test method.

### 2. Washing

The samples are washed using typical standard cotton wash cycle at 40°C, with Ariel color detergent. Possible de-lamination of coating from the samples is then visually checked.

### 3. Measurement of plasticizers weight loss

The weight loss has been measured according to the ASTM D 1239-98 method. The samples (5cm x 5cm) are conditioned for 24 hours in the climatic room (23°C, 50% relative humidity) and weighed. They are then immersed either in an excess 300ml of tap water or tap water and detergent (conc. = 7.5 g/l) with a magnetic stirrer. After 24 hours, the samples are washed from possible detergent residuals by soaking in distilled water, then left 24 hours in the climatic room and weighed again. The percentage of weight loss is computed as follows:

Weight loss % = [(Final weight - Initial weight)/ (Initial weight)] *100.

## EXAMPLES

*Example 1: Manufacturing of a moisture vapour permeable layer formed from a polymeric composition without plasticizer in solution.*

To a mixture of 100 parts of UCB Ucecoat UD 214, 5 parts of cross linker UCB Ucecoat T 376 B and 1 part of Ucecoat T202 B (acidic catalyst), is added 10 parts of Toluene. The resulting solution is then coated on a release paper, by adjusting the coating head so that a dry coating weight of 20 microns is achieved. This solution is then dried at 80°C for 1.5 minutes, and the resulting film is cured at 150°C for 1.5 minutes.
The MVTR measured as described in the test methods, is at 600 g/m$^2$.day.

*Example 2: Manufacturing of a moisture vapour permeable layer formed from a polymeric composition containing 35% plasticizer Pluronic PE 6400, in solution*

To a mixture of 100 parts of UCB Ucecoat UD 214, 27 parts of Pluronic PE 6400, 5 parts of cross linker Uceocoat T 376 B and 1 part of Uceacoat T 202 B, is added 10 parts of Toluene. The resulting solution is then coated on a release paper, by adjusting the coating head so that a dry coating weight of 20 microns is achieved. This solution is then dried at 80°C for 1.5 minutes, and the resulting film is cured at 150°C for 1.5 minutes.
The MVTR is measured at 1350 g/m$^2$.day.
The plasticizer weight loss (extractability) is measured as described in the test methods, at 20% while the plasticizer (35%) is usually completely soluble.

*Example 3: Manufacturing of a moisture vapour permeable layer formed from a polymeric composition, according to example 2, with higher content of cross-linking agent*

To a mixture of 100 parts of UCB Ucecoat UD 214, 27 parts of Pluronic PE 6400, 8 parts of cross linker Ucecoat T 376 B and 2 parts of Ucecoat T 202 B, is added 10 parts of Toluene. The resulting solution is then coated on a release paper, by adjusting the coating head so that a dry coating weight of 20 microns is achieved. This solution is then dried at 80°C for 1.5 minutes, and the resulting film is cured at 150°C for 1.5 minutes.
The MVTR is measured at 1350 g/m$^2$.day.
The extractability value is measured at 10% while the plasticizer (35%) is completely soluble.

*Example 4: Manufacturing of a moisture vapour permeable layer formed from a polymeric composition containing PEG 6000 as a plasticizer, in solution*

A PEG emulsion is formed by slowly adding 80 parts of butan-2-one solution containing 2 parts of Pluronic 8100, to 20 parts of PEG 6000 melted at 70°C.
To 100 parts of UCB Ucecoat UD 214, is added 135 parts of PEG emulsion made in 1, 5 parts of cross linker Ucecoat T 376 B and 1 part of Ucecoat T 202 B. The resulting solution is then coated on a release paper, by adjusting the coating head so that a dry coating weight of 20 microns is achieved. This solution is then dried at 80°C for 1.5 minutes, and the resulting film is cured at 150°C for 1.5 minutes.
The MVTR is measured at 1300 g/m$^2$.day.
The extractability value is measured at 21% both in water and soapy water while the unreacted plasticizer (30%) is completely soluble.

*Example 5: Coating of a moisture vapour permeable layer formed from a polymeric composition onto a substrate*

A layer of UCECOAT AB7300 is coated on a release paper at 20-25 g/m$^2$, and the system is dried at 100°C for 3 minutes. Then, a layer of the composition of example 4 is applied on top of the first layer at 20-25 g/m$^2$, and the system is dried at 100°C for 3 minutes. On this wet layer, A4 sized swatches of a polyester fabric are put on and pressed by using a rubber roll. The material is dried at 80°C for 1.5 minutes and then cured at 150°C for 1.5 minutes.
The aforementioned coated fabric sample is then subjected to 5 successive washing at 40°C, and the extractability test revealed a weight loss of only 0.5%.

*Example 6: Manufacturing of a moisture vapour permeable layer formed from a polymeric composition, in waterborne system, without cross-linker*

To 180 parts of Sancure 200012, a polyether aliphatic waterborne polyurethane available from Noveon Inc, is mixed 20 parts of Diacetin and 20 parts of PEG 2000. This polymeric composition is then processed into a 100 microns film, dried at 60°C for 3 minutes and then fully dried at room temperature for 24 hours. However, due to a alck of reaction between the PEG and the polymer, the PEG crystallizes into the polymeric matrix, and the resulting film remains fragile. Measurements of MVTR are consequently rendered difficult.

*Example 7: Manufacturing of a moisture vapour permeable layer formed from a of polymeric composition, in waterborne system, with a cross-linker*

To a mixture of 180 parts of Sancure 200012, 20 parts of Diacetin, and 20 parts of PEG 2000, is added 5 parts of Citric acid. This polymeric composition is then processed into a 100 microns film, dried at 60°C for 3 minutes and then fully dried at room temperature for 24 hours. The resulting film, this time, remains elastic and strong. The PEG does not crystallize in the polymeric matrix and the film, which highlights a good reaction between the PEG and the polymer.
The MVTR value is measured at 1000 g/m$^2$ day, at 100 microns.

**Claims**

1. A polymeric composition comprising the reaction product of:

    a) a thermoplastic polymer or blend of thermoplastic polymers, and
    b) a hydrophilic plasticizer or blend of hydrophilic plasticizers,

    **characterized in that** at least one of said hydrophilic plasticizers is covalently bonded with at least one of said thermoplastic polymers.

2. A polymeric composition according to claim 1 wherein said thermoplastic polymer or blend of thermoplastic polymers are selected from the group consisting of polyurethanes, poly-ether-amides, poly-ether-esters, poly-ether-ester-amides, polyethylene-acrylic acid copolymers, styrene-maleic anhydride copolymers, polyamides, polyesters, or mixtures thereof.

3. A polymeric composition according to claims 1-2 wherein said hydrophilic plasticizers comprise at least one functional group that forms a covalent bonding with said thermoplastic polymer.

4. A polymeric composition according to claim 3 wherein said functional group is selected from hydroxyls, amines, epoxy, or olefinically or acetylenically unsaturated groups.

5. A polymeric composition according to claims 1-4 wherein said hydrophilic plasticizers are selected from the group consisting of monomeric or polymeric alcohols, polyvalent alcohols, monomeric or polymeric amines or polyvalent amines.

6. A polymeric composition according to claim 5 wherein said hydrophilic plasticizers are selected from the group consisting of polyethylene glycol, polypropylene glycol, polytetramethylene glycol and copolymers of thereof.

7. A polymeric composition according to claims 1-6 wherein said composition comprises an additional component which comprise at least two reactive groups capable of bridging said thermoplastic polymer and said hydrophilic plasticizer.

8. A polymeric composition according to claims 1-7 wherein said hydrophilic plasticizer is covalently bonded to said thermoplastic polymer forming a 3-dimensional network.

9. A polymeric composition according to claims 1-8 wherein said composition comprises a cross-linking agent.

10. A polymeric composition according to claim 9 wherein said cross-linking agent is aminoplast or phenoplast resin.

11. A polymeric composition according to claims 1-10 wherein said composition comprises catalysts and/or activators.

12. A polymeric composition according to claims 1-11 wherein said composition comprises at least one additional hydrophilic plasticizer, which is not capable of chemically bonding with said thermoplastic polymer or blend of thermoplastic polymers.

13. A polymeric composition according to claims 1-12, said composition being prepared in presence of a solvent, either as a homogeneous solution, or as dispersion or emulsion wherein said thermoplastic polymers and/or said hydrophilic plasticizers are present as emulsions, or dispersions or in solution in said dispersion or emulsion.

14. A polymeric composition according to claim 13, said composition being prepared in waterborne system, by mixing either waterborne emulsions of polymers and plasticizers, or solutions of polymers and emulsions of plasticizer, or polymer waterborne emulsions and solutions of plasticizers.

15. A polymeric composition according to claims 1-12, said composition being prepared by hot melt techniques.

16. A polymeric composition according to claim 15 wherein said polymeric composition is in the molten state and has a viscosity of from 50 poise to 4000 poise at a frequency of 1 rad/s at processing temperature and a viscosity of less than 2000 poise at a frequency of 100 rad/s at processing temperature, in which processing temperature is between 70-190°C.

17. A moisture vapour permeable layer formed from the polymeric composition of any of the preceding claims, wherein said layer is liquid impervious and has a water vapour transmission rate (WVTR) of at least 600 g/m$^2$.day, preferably at least 800 g/m$^2$.day, more preferably at least 1000 g/m$^2$.day, and most preferably at least 1300 g/m$^2$.day, with a thickness of said layer or film of at least 20 □m, said moisture transmission rate measured according to the Moisture Vapour Transmission Test described herein.

18. A moisture vapour permeable, liquid impervious composite comprising at least one layer of claim 17 coated onto a substrate, said substrate being moisture vapour permeable.

19. A moisture vapour permeable, liquid impervious composite according to claim 18 wherein said substrate is a woven, knitted or non woven substrate.

20. A moisture vapour permeable, liquid impermeable article wherein said article comprises at least one moisture vapour permeable, liquid impervious layer or composite according to any of the preceding claims.

21. A process for making a composition according to claim 1, comprising the steps of:

a) providing a thermoplastic polymer or blend of thermoplastic polymers, and
b) providing a hydrophilic plasticizer or blend of hydrophilic plasticizers capable of covalently bonding with at least one of said thermoplastic polymers to form said polymeric composition.

22. A process according to claim 21,
wherein said thermoplastic polymers or blend of thermoplastic polymers are selected from the group consisting of polyurethanes, poly-ether-amides, poly-ether-esters, poly-ether-ester-amides, polyethylene-acrylic acid copolymers, styrene-maleic anhydride copolymers, polyamides, polyesters, or mixtures thereof, and
wherein said hydrophilic plasticizers are selected from the group consisting of monomeric or polymeric alcohols, polyvalent alcohols, monomeric or polymeric amines or polyvalent amines.

**European Patent Office**

**EUROPEAN SEARCH REPORT**

Application Number

EP 02 44 7152

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GB 2 157 703 A (SHIRLEY INST) 30 October 1985 (1985-10-30) * page 1, line 121 - page 2, line 33 * * examples * * claims * | 1-22 | A61L15/42 |
| A | EP 0 963 837 A (PROCTER & GAMBLE) 15 December 1999 (1999-12-15) * paragraph [0021] * * claims * | 1-22 | |

TECHNICAL FIELDS SEARCHED (Int.Cl.7)

A61L

The present search report has been drawn up for all claims

| Place of search | Date of completion of the search | Examiner |
|---|---|---|
| THE HAGUE | 6 February 2003 | Thornton, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 02 44 7152

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2003

| Patent document cited in search report | | Publication date | Patent family member(s) | | Publication date |
|---|---|---|---|---|---|
| GB 2157703 | A | 30-10-1985 | NONE | | |
| EP 0963837 | A | 15-12-1999 | EP | 0963837 A1 | 15-12-1999 |
| | | | AU | 3842099 A | 30-12-1999 |
| | | | CA | 2332913 A1 | 16-12-1999 |
| | | | EP | 1085979 A1 | 28-03-2001 |
| | | | WO | 9964237 A1 | 16-12-1999 |

EPO FORM P0459

For more details about this annex : see Official Journal of the European Patent Office, No. 12/82